# EUROPÄISCHE PATENTANMELDUNG

(11) **EP 2 756 867 A1**
(43) Veröffentlichungstag der Anmeldung: **23.07.2014**
(21) Anmeldenummer: 14000055.5
(22) Anmeldetag: 08.01.2014
(51) Int. Cl.: A61N 1/375, A61N 1/39, A61N 1/362

(54) **Externer Herzschrittmacher mit einer über einen Steckverbinder angeschlossenen, temporär mit einem Herzen verbindbaren Elektrode**

(30) Priorität: 22.01.2013 DE 102013001021
(71) Anmelder: OSYPKA AG., 79618 Rheinfelden-Baden (DE)
(72) Erfinder: Göttsche, Thorsten, 79618 Rheinfelden (DE); Virnich, Astrid, 79618 Rheinfelden (DE); Gräfe, Maik, 79618 Rheinfelden (DE)
(74) Vertreter: Rummler, Felix

(57) **Zusammenfassung**

Ein externer Herzschrittmacher (1) weist wenigstens eine temporär mit dem Herzen (5) verbindbare Elektrode (2, 2a, 2b, 2c, 2d) auf, an deren distalen Ende (3) ein ein Gewebe (4) des Herzens (5) kontaktierender Wirkbereich (6) und an deren proximalen Ende (7) wenigstens ein Kontaktstecker (8, 8a) angeordnet sind. Die Elektrode (2, 2a, 2b, 2c, 2d) ist für die Übertragung von Stimulations- und/oder Defibrillations- und/oder Kardioversionsimpulsen geeignet und zu diesem Zweck in eine Steckerbuchse (9) am Herzschrittmacher (1) einsteckbar. Dabei weist der Kontaktstecker (8) einen Kontaktbereich (11) an seinem Steckerteil (19) und einen zweiten Kontaktbereich (12) an seinem Steckerteil (20) auf. Die Steckerbuchse (9) ist zu dem Kontaktstecker (8) passend ausgebildet und weist die beiden Gegenkontakte (13) und (14) auf, von denen der erste Gegenkontakt (13) zur Übertragung von Stimulationsimpulsen und der zweite Gegenkontakt (14) zur Übertragung von Defibrillations- und/oder Kardioversionsimpulsen auf den Kontaktstecker (8) eingerichtet sind. In eingesteckter Gebrauchsstellung gelangt nur der Kontaktbereich (11) an dem Steckerteil (19) mit dem für die Übertragung der Defibrillations- und/oder Kardioversionsimpulse vorgesehenen Gegenkontakt (14) in Berührung, sodass kein Defibrillations- und/oder Kardioversionsimpuls auf Elektroden, die für eine Defibrillation und/oder Kardioversion ungeeignet sind und lediglich einen einstufigen Kontaktstecker (8a) aufweisen, übertragen werden kann (Fig. 3).

## Beschreibung

Die Erfindung betrifft einen externen Herzschrittmacher mit wenigstens einer temporär mit dem Herzen verbindbaren Elektrode, an deren distalen Ende zumindest ein ein Gewebe des Herzens eines Patienten in Gebrauchsstellung kontaktierender Wirkbereich angeordnet ist, wobei die Elektrode in Gebrauchsstellung mit ihrem proximalen Ende über wenigstens einen Steckverbinder, bestehend aus zwei Teilen, nämlich aus einem Kontaktstecker und aus einer Steckerbuchse, zur Übertragung von Stimulationsimpulsen von dem externen Herzschrittmacher auf das Herz des Patienten an den externen Herzschrittmacher anschließbar oder angeschlossen ist, und wobei die Elektrode zumindest an ihrem das Gewebe des Herzens kontaktierenden Wirkbereich eine für die Übertragung oder Abgabe eines Defibrillations- und/oder Kardioversionsimpulses ausreichend große Oberfläche hat.

Ein vergleichbarer Herzschrittmacher mit wenigstens einer temporär mit einem Herzen verbindbaren Elektrode ist beispielsweise aus der US 5919213 A und aus der DE 20 2010 011 244 U1 bekannt.

Ein derartiger externer Herzschrittmacher wird insbesondere nach operativen Eingriffen am menschlichen Herzen zur Unterstützung der Herzfunktion verwendet. Dabei kann er als reiner Schrittmacher fungieren und Stimulationsimpulse auf das Herz abgeben, um eine bestimmte Pulsfrequenz oder einen bestimmten Herzrhythmus vorzugeben und/oder möglicherweise gefährliche Herzrhythmusstörungen zu vermeiden.

In akuten Notfällen, beispielsweise wenn der Patient eine gefährliche Herzrhythmusstörung, insbesondere ein Kammerflimmern, erleidet, kann es notwendig sein, auch einen Defibrillations- und/oder Kardioversionsimpuls auf das Herz abzugeben, um die Herzrhythmusstörung zu beseitigen.

Wenn in einem solchen Notfall aber eine nur für die Schrittmacherstimulation geeignete Elektrode verwendet wird, bedarf es für die Defibrillation und/oder Kardioversion einer zusätzlichen Defibrillations- und/oder Kardioversionsvorrichtung.

Aufgabe der Erfindung ist daher, einen externen Herzschrittmacher zu schaffen, der einerseits sowohl für eine Stimulation als auch für eine Defibrillation und/oder Kardioversion geeignet ist, bei dem andererseits jedoch die Abgabe von Defibrillations- und/oder Kardioversionsimpulsen auf eine für die Defibrillation und/oder Kardioversion ungeeignete Elektrode unterbunden ist.

Zur Lösung dieser Aufgabe wird bei einem eingangs definierten externen Herzschrittmacher vorgeschlagen, dass der Kontaktstecker des Steckverbinders einen ersten und einen zweiten elektrischen Kontaktbereich und die Steckerbuchse des Steckverbinders, dazu passend, einen ersten und einen zweiten elektrischen Gegenkontakt aufweisen, dass die beiden Kontaktbereiche oder die beiden Gegenkontakte des an dem externen Herzschrittmacher angeordneten Teils des Steckverbinders unterschiedlich beschaltbar oder beschaltet sind, sodass der eine der beiden Kontaktbereiche oder Gegenkontakte zur Übertragung von Stimulationsimpulsen und der andere der beiden Kontaktbereiche oder Gegenkontakte zur Übertragung von Defibrillations- und/oder Kardioversionsimpulsen auf die Elektrode ausgebildet sind, und dass der externe Herzschrittmacher mit einem Defibrillator und/oder Kardioverter verbunden ist und/oder einen Defibrillator und/oder Kardioverter enthält und/oder zur Abgabe von Defibrillations- und/oder Kardioversionsimpulsen eingerichtet ist. So ist es möglich, den externen Herzschrittmacher sowohl zur Stimulation als auch zur in Akutfällen möglicherweise notwendigen Defibrillation und/oder Kardioversion des Herzens zu verwenden.

Es ist möglich, dass die Steckerbuchse des wenigstens einen Steckverbinders an dem proximalen Ende der Elektrode und der Kontaktstecker an dem externen Herzschrittmacher vorgesehen sind.

Bei einer bevorzugten Ausführungsform der Erfindung sind jedoch der Kontaktstecker des wenigstens einen Steckverbinders an dem proximalen Ende der Elektrode und die Steckerbuchse des Steckverbinders an dem externen Herzschrittmacher angeordnet.

Dabei können die beiden elektrischen Gegenkontakte der Steckerbuchse derart unterschiedlich beschaltbar oder beschaltet sein, dass der eine der beiden Gegenkontakte zur Übertragung von Stimulationsimpulsen und der andere der beiden Gegenkontakte zur Übertragung von Defibrillations- und/oder Kardioversionsimpulsen jeweils über den entsprechenden Kontaktbereich des Kontaktsteckers auf die Elektrode eingerichtet ist.

Zur Übertragung von Defibrillations- und/oder Kardioversionsimpulsen weisen nur auch für die Kardioversion und/oder Defibrillation geeignete Elektroden wenigstens einen erfindungsgemäßen Kontaktstecker mit zwei Kontaktbereichen auf, sodass die Defibrillations- und/oder Kardioversionsimpulse von dem Herzschrittmacher nur auf eine Elektrode mit einem solchen Kontaktstecker abgegeben werden können.

Herkömmliche Elektroden, die nur für die Stimulation geeignet sind, können, aufgrund ihres gewöhnlichen Kontaktsteckers, nicht in Berührung mit dem Gegenkontakt für die Übertragung von Defibrillation- und/oder Kardioversionsimpulsen der Steckerbuchse gelangen, sodass eine Übertragung von Defibrillations- und/oder Kardioversionsimpulsen auf eine mit einem gewöhnlichen Kontaktstecker versehene Elektrode vermieden werden kann. Dadurch kann die Gefahr einer Verletzung des Herzens aufgrund einer Defibrillations- und/oder Kardioversionsimpulsabgabe über eine dafür ungeeignete Elektrode verringert oder gar ausgeschlossen sein.

Die Fehlbeschaltung einer aufgrund der Form ihres Kontaktsteckers ungeeigneten Elektrode kann besonders effektiv vermieden werden, wenn die beiden unterschiedlich beschaltbaren oder beschalteten Gegenkontakte der Steckerbuchse unterschiedlich und die beiden Kontaktbereiche des Kontaktsteckers passend zu den Gegenkontakten geformt sind und/oder wenn die beiden Kontaktbereiche des Kontaktsteckers als zwei zu den beiden Gegenkontakten der Steckerbuchse passende Steckerteile ausgebildet sind. Die Steckerbuchse kann zumindest in ihrem Inneren also beispielsweise zweistufig und der Kontaktstecker dazu passend oder komplementär ausgestaltet sein.

Auf diese Weise ist es möglich, dass nur ein entsprechend geformter Kontaktstecker so in die Steckerbuchse eingesteckt werden kann, dass seine beiden Kontaktbereiche die beiden Gegenkontakte der Steckerbuchse berühren, um neben Stimulationsimpulsen bei Bedarf auch Defibrillations- und/oder Kardioversionsimpulse auf eine an den Kontaktstecker angeschlossene Elektrode übertragen zu können.

Mit gewöhnlichen Kontaktsteckern, die nicht die beiden passend zu beiden Gegenkontakten der Steckerbuchse geformten Kontaktbereiche und/oder nicht die beiden als Kontaktbereiche vorgesehenen Steckerteile aufweisen, kann eine versehentliche Kontaktierung des Gegenkontakts für die Abgabe von Defibrillations- und/oder Kardioversionsimpulsen unterbunden sein.

So ist es möglich Elektroden, die ausschließlich für die Stimulation, aber nicht für die Defibrillation und/oder Kardioversion vorgesehen und/oder geeignet sind, mit gewöhnlichen Kontaktsteckern auszurüsten, die zwar den Gegenkontakt der Steckerbuchse für die Abgabe von Stimulationsimpulsen kontaktieren, den Gegenkontakt der Steckerbuchse für die Abgabe der Defibrillations- und/oder Kardioversionsimpulse aber auch in eingesteckter Gebrauchsstellung nicht erreichen.

Eine versehentliche Übertragung eines Defibrillationsund/oder Kardioversionsimpulses auf derartige Elektroden'kann somit effektiv vermieden werden.

Zur unterschiedlichen Gestaltung der Gegenkontakte der Steckerbuchse können der erste und der zweite Gegenkontakt der Steckerbuchse in Einsteckrichtung des Kontaktsteckers hintereinander angeordnet sein und zwei unterschiedlich große Innenquerschnitte aufweisen, wodurch ein Kontaktstecker, mit dem keine Defibrillations- und/oder Kardioversionsimpulse übergeleitet werden dürfen und der nur einen zu dem ersten Gegenkontakt passenden Kontaktbereich aufweist, nicht falsch gesteckt werden kann.

Dazu kann es zweckmäßig sein, wenn der erste Gegenkontakt der Steckerbuchse einen größeren Innenquerschnitt als der zweite Gegenkontakt hat und als Kontaktfläche zur Übertragung von Stimulationsimpulsen auf die Elektrode ausgebildet ist und wenn der zweite, in Einsteckrichtung des Kontaktsteckers benachbarte Gegenkontakt der Steckerbuchse einen kleineren in dem Querschnitt als der erste Gegenkontakt hat und als Kontaktfläche zur Übertragung von Defibrillations- und/oder Kardioversionsimpulsen auf die Elektrode ausgebildet sind.

Dabei ist es natürlich auch denkbar, dass der in Einsteckrichtung des Kontaktsteckers erste Gegenkontakt der Steckerbuchse als Kontaktfläche zur Übertragung von Defibrillationsund/oder Kardioversionsimpulsen und der in Einsteckrichtung des Kontaktsteckers zweite Gegenkontakt als Kontaktfläche zur Übertragung von Stimulationsimpulsen auf die Elektrode ausgebildet ist.

In beiden Fällen sind jedoch der erste und der zweite Gegenkontakt im Inneren der Steckerbuchse derartig elektrisch voneinander getrennt oder isoliert, dass nur der für die Stimulation vorgesehene Gegenkontakt den Stimulationsimpuls und nur der für die Defibrillation und/oder Kardioversion vorgesehene Gegenkontakt den Defibrillations- und/oder Kardioversionsimpuls übertragen kann, also kein Überspringen der Impulse auf den jeweils anderen Gegenkontakt möglich ist.

Dabei kann der erste Gegenkontakt mit dem größeren Innenquerschnitt in Gebrauchsstellung an eine Eintrittsöffnung der Steckerbuchse für den Kontaktstecker angrenzend angeordnet sein und der zweite Gegenkontakt mit dem kleineren Innenquerschnitt in Einsteckrichtung des Kontaktsteckers dahinter folgen, also tiefer im Inneren der Steckerbuchse liegen.

Um den in Gebrauchsstellung in die Steckerbuchse eingesteckten Kontaktstecker zu sichern und vor einem unbeabsichtigten Herausziehen zu schützen, kann die Steckerbuchse im ersten Gegenkontakt eine Spannzangenform und/oder einen geschlitzten Bereich mit einzelnen Fingern, die durch eine Überwurfmutter und einen konischen Bereich zusammendrückbar sind und/oder im zweiten Gegenkontakt Kontaktfedern aufweisen.

Es ist aber auch möglich, dass ein in den zweiten Gegenkontakt der Steckerbuchse passender, im Querschnitt dünnerer erster Teil des Kontaktsteckers geschlitzt und/oder elastisch verformbar ist und dass der Kontaktstecker in Gebrauchsstellung in der Steckerbuchse mit wenigsten einem seiner Kontaktbereiche kraftschlüssig gehalten und/oder festgeklemmt ist.

Zur Sicherung des Kontaktsteckers in seiner eingesteckten Gebrauchsstellung in der Steckerbuchse, ist es auch möglich, den Kontaktstecker mittels einer Schraube und/oder einer Schraubverbindung an der Steckerbuchse zu befestigen. Die Schraube kann dabei an einem Griffstück des Kontaktsteckers und/oder an einem oder an beiden Kontaktbereichen des Kontaktsteckers angreifen.

Um einen guten Sitz des Kontaktsteckers in seiner eingesteckten Gebrauchsstellung und eine gute elektrische Konnektivität zur ermöglichen, können die die beiden Kontaktbereiche des Kontaktsteckers bildenden und zu den beiden Gegenkontakten der Steckerbuchse passenden Steckerteile in Einsteckrichtung des Kontaktsteckers hintereinander angeordnet und mit derart unterschiedlichen Querschnitten ausgebildet sein, dass der zweite Steckerteil des Kontaktsteckers, der in eingesteckter Gebrauchsstellung den ersten Gegenkontakt der Steckerbuchse kontaktiert, den in axialer Richtung dahinter angeordneten, in eingesteckter Gebrauchsstellung den zweiten Gegenkontakt der Steckerbuchse kontaktierenden ersten Steckerteil des Kontaktsteckers zumindest bereichsweise radial überragt. So kann der Kontaktstecker für die für eine Defibrillation und/oder Kardioversion geeignete Elektrode ebenfalls eine zweistufige und zu der Steckerbuchse passende Form aufweisen.

Beispielsweise können der erste und der zweite Steckerteil des Kontaktsteckers jeweils einen geschlossenen und/oder kreisrunden Querschnitt haben oder wenigstens einer der beiden Steckerteile des Kontaktsteckers einen von einer Kreisform abweichenden Querschnitt, der insbesondere oval oder dreieckig oder viereckig oder mehreckig oder kreisförmig mit wenigstens einer Abflachung ist. Die Steckerbuchse kann folglich als Schloss und der Kontaktstecker als Schlüssel fungieren, um sicherzustellen, dass der für die Übertragung von Stimulationsimpulsen vorgesehene Kontaktbereich des Kontaktsteckers nicht mit dem für die Abgabe von Defibrillationsund/oder Kardioversionsimpulsen ausgebildeten Gegenkontakt der Steckerbuchse in Berührung kommt.

Der Kontaktstecker kann aber auch derartig ausgestaltet sein, dass einer der beiden Steckerteile des Kontaktsteckers einen mehreckigen, insbesondere dreieckigen Querschnitt hat, also die Form eines Prismas aufweist, und der andere Steckerteil des Kontaktsteckers einen runden Querschnitt hat, also zylindrisch ist. Auch bei dieser Ausgestaltung des Kontaktsteckers und einer entsprechenden Ausgestaltung der Steckerbuchse kann eine Fehlbeschaltung der Elektrode vermieden werden.

Alternativ kann der zweite Steckerteil des Kontaktsteckers mit dem größeren Querschnitt einen kreisrunden Querschnitt aufweisen, während der erste Steckerteil des Kontaktsteckers mit dem kleineren Querschnitt eine beliebige Querschnittsform aufweist, wobei die größte Abmessung des ersten, kleineren Steckerteils von der Projektion des kreisrunden Querschnitts des zweiten, größeren Steckerteils des Kontaktsteckers umschlossen ist. Auch so kann, eine passend zu dem Kontaktstecker ausgestaltete Steckerbuchse vorausgesetzt, eine Fehlkontaktierung des Kontaktsteckers vermieden werden.

Um ein Einstecken des Kontaktsteckers in die Steckerbuchse zu erleichtern, können die Längsmittelachsen der beiden Steckerteile des Kontaktsteckers koaxial zueinander und/oder miteinander fluchtend angeordnet sein. Bei miteinander fluchtenden Längsmittelachsen der beiden Steckerteile des Kontaktsteckers, muss der Stecker zum Einstecken in die entsprechend gestaltete Steckerbuchse nicht ausgerichtet werden, sondern kann sich beim Einstecken in die Steckerbuchse selbst zentrieren und so besonders einfach in seine eingesteckte Gebrauchsstellung geführt werden.

Dies kann insbesondere dann von Vorteil sein, wenn der Kontaktstecker, beispielsweise in einer Notsituation, besonders schnell und besonders sicher in die Steckerbuchse an dem externen Herzschrittmacher eingesteckt werden muss.

Es ist aber auch denkbar, dass die Längsmittelachsen der beiden Steckerteile des Kontaktsteckers parallel, insbesondere versetzt zueinander verlaufend, angeordnet sind.

Die Elektrode kann dadurch zur bipolaren und/oder quattripolaren und/oder multipolaren Stimulation und/oder Defibrillation und/oder Kardioversion ausgebildet sein, dass sie wenigstens zwei oder vier oder mehr Pole aufweist und dass für jeden Pol der Elektrode je ein Steckverbinder, bestehend aus einem Kontaktstecker und aus einer Steckerbuchse, vorgesehen ist.

Die Elektrode kann also an ihrem proximalen Ende für jeden Pol je einen Kontaktstecker und der Herzschrittmacher für jeden Kontaktstecker wenigstens eine Steckerbuchse aufweisen, wobei zumindest eine der beiden Steckerbuchsen mit den beiden unterschiedlich beschaltbaren und unterschiedlich gestalteten Gegenkontakten und zumindest der in Gebrauchsstellung in dieser Steckerbuchse eingesteckte Kontaktstecker mit den beiden zu den beiden Gegenkontakten der Steckerbuchse passenden Steckerteilen als Kontaktbereiche ausgestattet sind.

Die Verwendung einer bipolaren oder einer quattripolaren anstatt einer monopolaren Elektrode kann zu bevorzugen sein, da bei einer bipolaren oder auch einer quattripolaren Elektrode auf eine anderweitig im oder am Patienten anbringbare weitere Elektrode verzichtet werden kann.

Insbesondere bei Elektroden, die zwei oder mehr Pole aufweisen, kann es für eine gute Überleitung der Stimulationsund/oder Defibrillations- und/oder Kardioversionsimpulse auf das Herz günstig sein, wenn die Elektrode an ihrem distalen Ende wenigstens einen zweiten das Gewebe des Herzens eines Patienten kontaktierenden Wirkbereich aufweist und/oder wenn an jedem Wirkbereich der Elektrode wenigstens ein Pol, insbesondere zwei Pole angeordnet sind.

Der Kontaktstecker kann bereits im Auslieferungszustand der Elektrode mit dieser verbunden und/oder nachträglich mit der Elektrode verbindbar und/oder an diese anschließbar sein. Wenn der Kontaktstecker nachträglich mit der Elektrode verbindbar und/oder an diese anschließbar ist, ist es möglich, den erfindungsgemäßen Kontaktstecker mit unterschiedlichen Elektroden, auch für die Defibrillation und/oder Kardioversion geeignete Elektroden einzusetzen und/oder diese bei Bedarf auszutauschen.

Die Elektrode kann als Herzdraht ausgebildet sein, der beispielsweise nach einem kardiochirurgischen Eingriff außen am Herzen befestigbar, insbesondere mit dem Gewebe des Herzens vernäht und/oder in diesem verankert, sein kann. Diese Art von Elektroden wird häufig nach kardiochirurgischen Eingriffen eingesetzt und zur postoperativen, vorübergehenden Stimulation des Herzens in Kombination mit einem externen Herzschrittmacher eingesetzt.

Die Elektrode kann aber auch als ein, insbesondere mit am proximalen Ende angeordnetem Kontaktstecker, intrakardial zuführbarer Katheter ausgebildet sein, der vorzugsweise von außen über ein Blutgefäß in das Innere des Herzens vorgeschoben wird, um dort das Herz zu stimulieren, zu defibrillieren und/oder zu kardiovertieren.

Anhand der Zeichnung sind nachfolgend mehrere Ausführungsbeispiele der Erfindung näher beschrieben. Die Figuren zeigen in teilweise schematisierter Darstellung:
- Figur 1:: eine schematisierte Darstellung eines externen Herzschrittmachers mit vier Steckerbuchsen, von denen eine im Schnitt dargestellt ist, und mit einer unipolaren Elektrode, die einen ein Gewebe des Herzens kontaktierenden Wirkbereich an ihrem distalen Ende und einen Kontaktstecker an ihrem dem externen Herzschrittmacher zugewandten, proximalen Ende aufweist, wobei der Kontaktstecker als Stufenstecker mit zwei unterschiedlichen Kontaktbereichen ausgebildet ist,
- Figur 2:: der in Figur 1 dargestellte externe Herzschrittmacher mit einer bipolaren Elektrode mit einem ein Gewebe des Herzens kontaktierenden Wirkbereich an ihrem distalen Ende und mit zwei Kontaktsteckern an ihrem proximalen Ende, wobei der eine Kontaktstecker nur einen Kontaktbereich aufweist und nur zur Übertragung von Stimulationsimpulsen und der andere Kontaktstecker zwei Kontaktbereiche aufweist, und daher auch zur Übertragung von Defibrillationsund/oder Kardioversionsimpulsen auf die Elektrode ausgebildet ist,
- Figur 3:: der in den Figuren 1 und 2 dargestellte externe Herzschrittmacher mit einer bipolaren Elektrode, die an ihrem proximalen Ende zwei zweistufige Kontaktstecker aufweist,
- Figur 4:: der in den Figuren 1 bis 3 dargestellte externe Herzschrittmacher mit einer quattripolaren Elektrode mit zwei implantierbaren Bereichen an ihrem distalen Ende und mit vier Kontaktsteckern an ihrem proximalen Ende, von denen zwei Kontaktstecker je zwei Kontaktbereiche aufweisen und als Stufenstecker ausgebildet sind,
- Figur 5:: der in den Figuren 1 bis 4 dargestellte externe Herzschrittmacher mit einer quattripolaren Elektrode mit zwei implantierbaren Bereichen an ihrem distalen Ende und mit vier Kotaktsteckern an ihrem proximalen Ende, wobei alle vier Kontaktstecker jeweils stufenförmig ausgebildet sind,
- Figur 6:: eine Darstellung eines erfindungsgemäßen zweistufigen Kontaktsteckers mit zwei Kontaktbereichen, wobei der Kontaktstecker eine Querbohrung für den Anschluss und die Befestigung einer Elektrode aufweist,
- Figur 7:: eine der Figur 6 ähnelnde Darstellung eines weiteren erfindungsgemäßen zweistufigen Kontaktsteckers mit zwei Kontaktbereichen, wobei der Kontaktstecker eine Längsbohrung für den Anschluss einer Elektrode aufweist,
- Figur 8:: eine im Längsschnitt dargestellte erfindungsgemäße Steckerbuchse mit einem nicht eingesteckten, erfindungsgemäßen zweistufigen Kontaktstecker, wobei im Inneren der Steckerbuchse zwei in Einsteckrichtung des Kontaktsteckers hintereinander angeordnete Gegenkontakte zu erkennen sind,
- Figur 9:: die in Figur 7 dargestellte Steckerbuchse mit dem in Figur 7 ebenfalls dargestellten Kontaktstecker in eingesteckter Gebrauchsstellung, wobei der eine Gegenkontakt der Steckerbuchse den einen der beiden Kontaktbereiche des Kontaktsteckers und der andere Gegenkontakt den anderen Kontaktbereich des Kontaktsteckers kontaktiert,
- Figur 10:: die in den Figuren 8 und 9 dargestellte Steckerbuchse mit einem herkömmlichen, einstufigen Kontaktstecker,
- Figur 11:: der in Figur 10 dargestellte einstufige Kontaktstecker in eingesteckter Gebrauchsstellung in der Steckerbuchse, wobei der Kontaktstecker mit seinem einen Kontaktbereich lediglich den ersten der beiden Gegenkontakte berührt,
- Figur 12:: eine vergrößerte Seitenansicht der in den Figuren 1 bis 5 sowie 7 bis 11 dargestellten Steckerbuchsen,
- Figur 13:: ein Längsschnitt durch die in Figur 12 dargestellte Steckerbuchse, wobei eine Spannzange für einen Kontaktstecker und die beiden unterschiedlich dimensionierten und unterschiedlich gestalteten Gegenkontakte zu erkennen sind, sowie
- Figur 14:: der in den Figuren 1 bis 5 dargestellte externe Herzschrittmacher mit einer als ein intrakardial zuführbarer Katheter ausgebildeten, in ihrer in ein Herz eines Patienten eingeführten Gebrauchsstellung befindlichen Elektrode mit zwei Kontaktsteckern an ihrem proximalen Ende.

Ein im Ganzen mit 1 bezeichneter externer Herzschrittmacher weist wenigstens eine temporär mit einem Herzen 5 verbindbaren Elektrode 2, 2a, 2b, 2c, 2d auf.

An einem distalen Ende 3 der Elektrode 2, 2a, 2b, 2c, 2d befindet sich wenigstens ein ein Gewebe 4 des Herzens 5 eines Patienten in Gebrauchsstellung der Elektrode 2, 2a, 2b, 2c, 2d kontaktierender Wirkbereich 6, über den elektrische Impulse von dem Herzschrittmacher 1 auf das Herz 5 abgegeben werden können.

Der Wirkbereich 6 wird mithilfe einer Herznadel 6a im Gewebe 4 des Herzens 5 positioniert, sodass er das Gewebe 4 des Herzens 5 unmittelbar berührt.

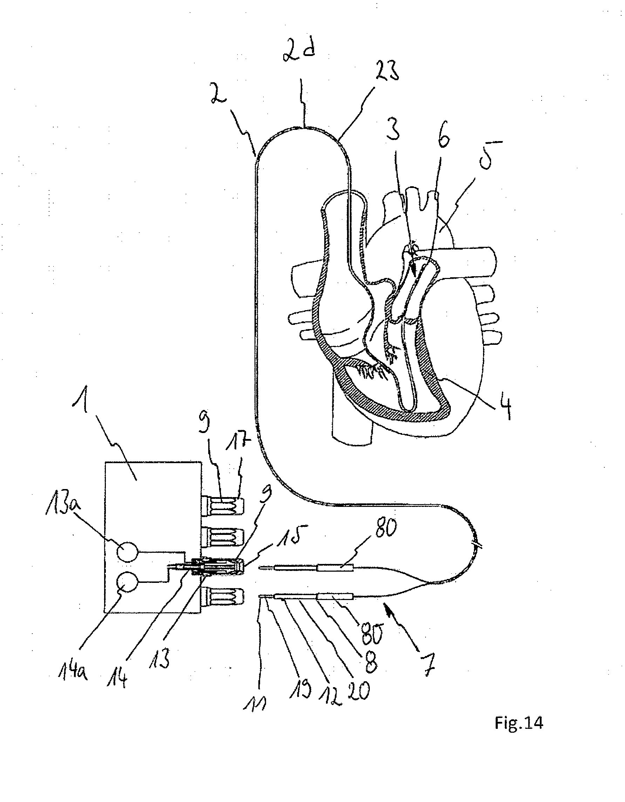

Bei einer anderen Ausgestaltung der Erfindung (vgl. Fig. 14) kontaktiert der Wirkbereich 6 ein oder das Gewebe 4 des Herzens 6 mittelbar, beispielsweise über eine elektrisch leitfähige Körperflüssigkeit, sodass eine direkte Berührung des Gewebes 4 zur Übertragung von elektrischen Impulsen auf das Herz 5 nicht notwendig ist.

Die Elektrode 2, 2a, 2b, 2c, 2d ist über wenigstens einen Steckverbinder, bestehend aus einem Kontaktstecker 8 und einer Steckerbuchse 9, mit dem externen Herzschrittmacher 1 verbunden. Die in den Figuren dargestellten Elektroden 2, 2a, 2b, 2c, 2d weisen dazu an ihren dem Herzschrittmacher 1 in Gebrauchsstellung zugewandten proximalen Enden 7 jeweils wenigstens einen Kontaktstecker 8 und 8a auf, der zum Anschluss der jeweiligen Elektrode 2, 2a, 2b, 2c und 2d an den externen Herzschrittmacher 1 und zur Übertragung von Stimulationsimpulsen von dem externen Herzschrittmacher 1 auf das Herz 5 des Patienten in die wenigstens eine an dem externen Herzschrittmacher 1 angeordnete Steckerbuchse 9 einsteckbar und in Gebrauchsstellung eingesteckt ist.

Bei einer anderen, nicht dargestellten Ausführung der Erfindung kann der wenigstens eine Kontaktstecker 8 und/oder 8a an dem externen Herzschrittmacher 1 und die wenigstens eine Steckerbuchse 9 an dem proximalen Ende 7 der Elektrode 2, 2a, 2b, 2c und 2d angeordnet sein.

Um die Handhabung der Kontaktstecker 8 und 8a, insbesondere beim Ein- und Ausstecken, zu erleichtern, weisen sämtlichen in den Figuren dargestellten Kontaktstecker 8, 8a jeweils einen Griff 80 auf.

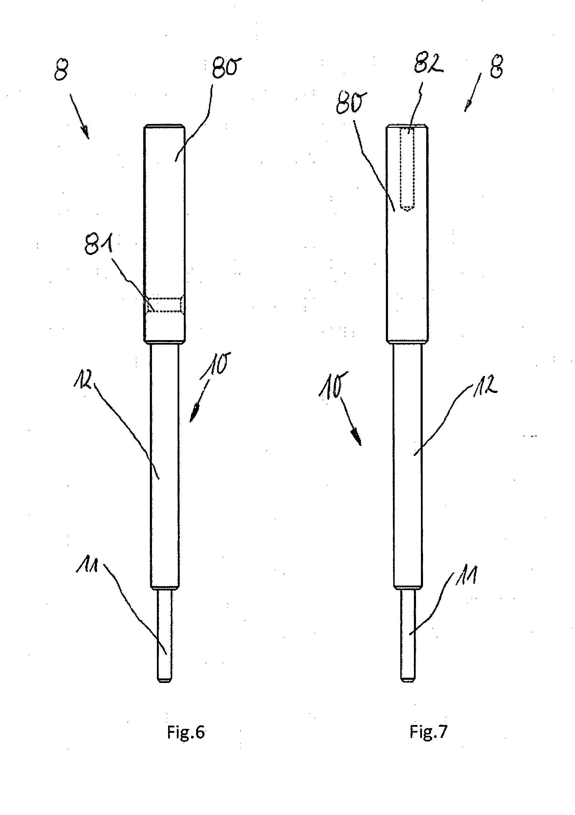

Die Figuren 6 und 7 zeigen mögliche Ausführungsformen des Kontaktsteckers 8. Für Elektroden 2, 2a, 2b, 2c, 2d, die noch keinen zweistufigen Kontaktstecker 8 aufweisen, können diese Kontaktstecker 8 auch als Adapter verwendet und nachträglich mit der Elektrode 2, 2a, 2b, 2c, 2d verbunden bzw. an diese angeschlossen werden.

Gemäß Figur 6 weist der Kontaktstecker 8 zum Anschluss einer Elektrode 2, 2a, 2b, 2c, 2d eine Querbohrung 81 auf, die den Griff 80 des Kontaktsteckers 8 durchsetzt und in der ein abisoliertes Leiterende der Elektrode 2, 2a, 2b, 2c, 2d platziert und befestigt werden kann.

Gemäß Figur 7 hat der Kontaktstecker 8 eine Längsbohrung 82 in seinem Griff 80, die der Aufnahme und des Anschlusses einer Elektrode 2, 2a, 2b, 2c, 2d an der Kontaktstecker 8 dient.

Die Befestigungsmechanismen, mit denen die Elektrode 2, 2a, 2b, 2c, 2d an dem jeweiligen Kontaktstecker 8 gehalten wird, sind nicht dargestellt, können aber beispielsweise durch ein Schlauchsegment gebildet werden.

Jede der dargestellten Elektroden 2, 2a, 2b, 2c, 2d hat zumindest einen in Gebrauchsstellung am Herzen 5 angreifenden Wirkbereich 6, der eine für die Übertragung oder Abgabe eines Defibrillations- und/oder Kardioversionsimpulses ausreichend große Oberfläche hat und über den die Stimulations- und/oder Defibrillations- und/oder Kardioversionsimpulse auf das Herz 5 übertragen werden.

Zur Positionierung des Wirkbereichs 6 im Gewebe des Herzens 5 weisen die Elektroden 2, 2a, 2b und 2c jeweils mindestens eine Herznadel 6a auf, die nach der Positionierung des Wirkbereichs 6 von der Elektrode 2, 2a, 2b, 2c abgetrennt, insbesondere abgeschnitten oder abgebrochen, werden kann.

Für die Übertragung von Stimulationsimpulsen und Defibrillations- und/oder Kardioversionsimpulsen auf die Elektrode 2, 2a, 2b, 2c, 2d hat der Kontaktstecker 8 in Gebrauchsstellung einen Kontakt 10 mit einem ersten und mit einem zweiten Kontaktbereich 11 und 12 und die an dem externen Herzschrittmacher 1 angeordnete Steckerbuchse 9 dazu passend zwei unterschiedliche beschaltbare Gegenkontakte 13 und 14.

Der Gegenkontakt 13 ist dabei zur Übertragung von Stimulationsimpulsen und der Gegenkontakt 14 zur Übertragung von Defibrillations- und/oder Kardioversionsimpulsen über den Kontaktstecker 8 auf die Elektrode 2, 2a, 2b, 2c, 2d ausgebildet.

Der Gegenkontakt 13 wird von einer Stromquelle 13a und der Gegenkontakt 14 von einer Stromquelle 14a versorgt.

Der externe Herzschrittmacher 1 ist zur Defibrillation und/oder Kardioversion eingerichtet und/oder weist zur Bereitstellung von Defibrillationsimpulsen und/oder Kardioversionsimpulsen einen in den Figuren nicht dargestellten Defibrillator und/oder Kardioverter auf oder ist mit einem solchen verbunden.

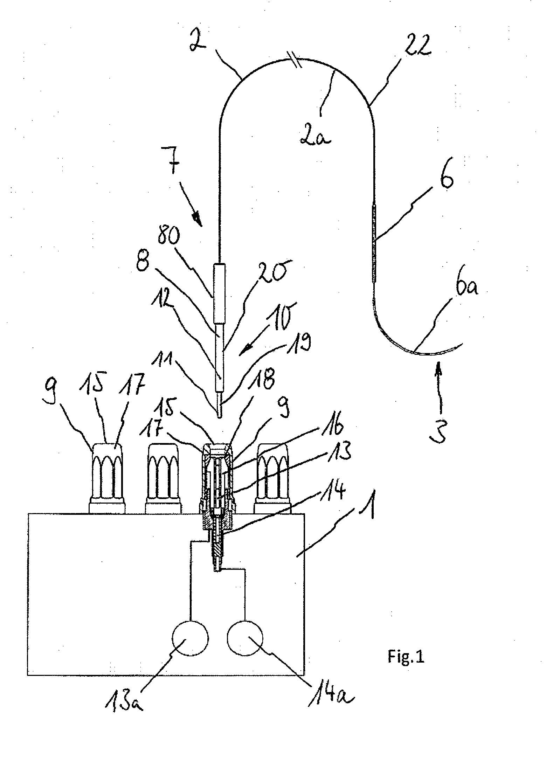

Wie die Figuren 1 bis 5 sowie die Figuren 7 und 8 zeigen, sind die beiden unterschiedlich beschaltbaren oder beschalteten Gegenkontakte 13 und 14 der Steckerbuchse 9 unterschiedlich geformt. Dazu sind der erste und der zweite Gegenkontakt 13 und 14 der Steckerbuchse 9 in Einsteckrichtung des Kontaktsteckers 8 hintereinander angeordnet und weisen unterschiedlich große Innenquerschnitte auf.

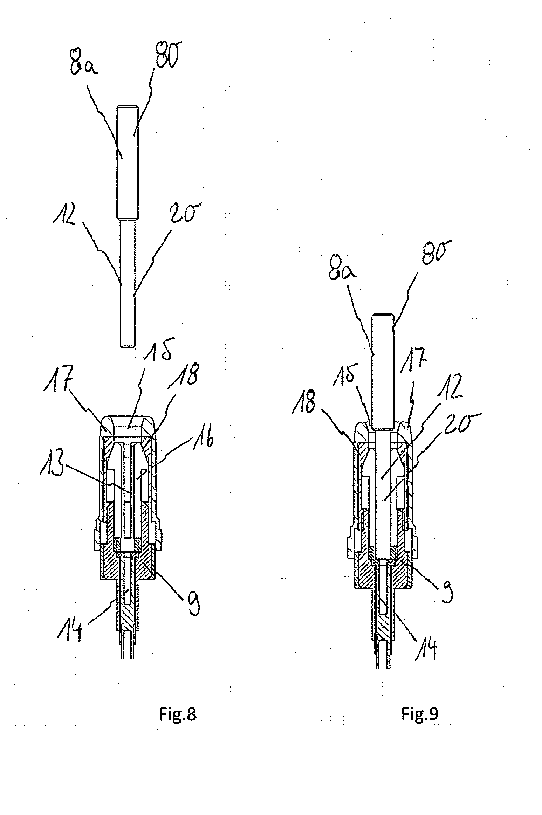

Wie insbesondere die Figuren 7 und 8 zeigen, hat dabei der erste Gegenkontakt 13 der Steckerbuchse 9 einen größeren Innenquerschnitt als der zweite Gegenkontakt 14, der überdies auch noch tiefer im Inneren der Steckerbuchse 9 angeordnet ist.

Der erste Gegenkontakt 13 ist, wie bereits ausgeführt, als Kontaktfläche zur Übertragung von Stimulationsimpulsen auf die Elektrode 2, 2a, 2b, 2c, 2d ausgebildet, während der zweite, in Einsteckrichtung des Kontaktsteckers 8 benachbarte Gegenkontakt 14 der Steckerbuchse 9 als Kontaktfläche zur Übertragung von Defibrillations- und/oder Kardioversionsimpulsen auf die Elektrode 2, 2a, 2b, 2c, 2d ausgebildet ist.

In einer nicht dargestellten Ausführungsform kann aber auch der zweite Gegenkontakt 14 der Steckerbuchse 9 zur Übertragung von Stimulationsimpulsen und der erste Gegenkontakt 13 der Steckerbuchse 9 zur Übertragung von Defibrillationsund/oder Kardioversionsimpulsen auf die Elektrode 2, 2a, 2b, 2c, 2d und somit auf das Herz 5 ausgebildet sein.

Dabei grenzt der erste Gegenkontakt 13 mit dem größeren Innenquerschnitt in Gebrauchsstellung an eine Eintrittsöffnung 15 der Steckerbuchse 9 für den Kontaktstecker 8 an, während der zweite Gegenkontakt 14 mit dem kleineren Innenquerschnitt in Einsteckrichtung des Kontaktsteckers 8 sich dahinter anschließt. Der zweite Gegenkontakt 14 ist also tiefer im Inneren der Steckerbuchse 9 als der erste Gegenkontakt 13 angeordnet.

Um den Kontaktstecker 8 in seiner Gebrauchsstellung festlegen zu können und ein unbeabsichtigtes Herausziehen des Kontaktsteckers aus seiner Gebrauchsstellung zu vermeiden, weist die Steckerbuchse im ersten Gegenkontakt 13 eine Spannzange 16 auf, die durch eine Überwurfmutter 17 und/oder ein Überwurfteil mit einem Innengewinde und einen konischen Bereich 18, den die Überwurfmutter 17 beim Anziehen beaufschlagt, zusammendrückbar ist, so dass der Kontaktstecker 8 in seiner Gebrauchsstellung in der Steckerbuchse 9 fixierbar ist.

In einer nicht dargestellten Ausführungsform kann die Steckerbuchse 9 aber auch im zweiten Gegenkontakt 14 Kontaktfedern aufweisen und/oder ein in den zweiten Gegenkontakt 14 der Steckerbuchse 9 passender, im Querschnitt dünnerer erster Teil des Kontaktsteckers 8 geschlitzt und/oder elastisch verformbar sein, so dass auch bei dieser Ausgestaltung der Steckerbuchse 9 der Kontaktstecker 8 in Gebrauchsstellung in der Steckerbuchse 9 mit wenigstens einem seiner Kontaktbereiche 11 oder 12 kraftschlüssig gehalten und/oder festgeklemmt ist.

Die beiden Kontaktbereiche 11 und 12 des Kontaktsteckers 8 sind als zwei zu den beiden Gegenkontakten 13 und 14 der Steckerbuchse 9 passende, in Einsteckrichtung hintereinander angeordnete Steckerteile 19 und 20 ausgebildet und haben unterschiedliche Querschnitte bzw. Durchmesser, so dass der zweite Steckerteil 20 des Kontaktsteckers 8, der in eingesteckter Gebrauchsstellung den ersten Gegenkontakt 13 der Steckerbuchse 9 kontaktiert, den in axialer Richtung dahinter angeordneten, in eingesteckter Gebrauchsstellung den zweiten Gegenkontakt 14 der Steckerbuchse 9 kontaktierenden ersten Steckerteil 19 des Kontaktsteckers 8 zumindest bereichsweise radial überragt.

Im vorliegenden Ausführungsbeispiel haben der erste und der zweite Steckerteil 19 und 20 jeweils einen kreisrunden Querschnitt. Es ist aber auch möglich, dass wenigstens einer der beiden Steckerteile 19 oder 20 einen von einer Kreisform abweichenden Querschnitt hat, der insbesondere oval oder dreieckig oder viereckig oder mehreckig oder kreisförmig mit wenigstens einer Abflachung ist.

In einer anderen, ebenfalls nicht dargestellten Ausführungsform des Kontaktsteckers 8 kann einer der beiden Steckerteile 19 oder 20 einen mehreckigen, insbesondere dreieckigen Querschnitt haben, also die Form eines Prismas aufweisen, und der andere Steckerteil 19 oder 20 einen runden Querschnitt haben, also zylindrisch sein.

Um die Fehlkontaktierung und/oder die Fehlbelegung der Elektrode 2, 2a, 2b, 2c, 2d effektiv zu vermeiden, ist bei einer weiteren nicht dargestellten Ausführungsform des Kontaktsteckers 8 vorgesehen, dass der zweite Steckerteil 20 mit dem größeren Querschnitt einen kreisrunden Querschnitt aufweist, während der erste Steckerteil 19 mit dem kleineren Querschnitt eine beliebige Querschnittsform aufweist, wobei die größte Abmessung des ersten, kleineren Steckerteils 19 von der Projektion des kreisrunden Querschnitts des zweiten, größeren Steckerteils 20 umschlossen ist.

Die Längsmittelachsen der beiden Steckerteile 19 und 20 des Kontaktsteckers 8 sind koaxial zueinander und miteinander fluchtend angeordnet. Dies erleichtert ein Einführen des Kontaktsteckers 8 in die Steckerbuchse 9, da sich der Kontaktstecker 8 aufgrund der Koaxialität seiner beiden Steckerteile 19 und 20 und der passend dazu ausgebildeten Steckerbuchse 9 mit ihren Gegenkontakten 13 und 14 während des Einführens selbst zentriert, also nicht von Hand erst in eine für das Einführen richtige Orientierung verdreht werden muss.

Die Figuren 8 und 9 veranschaulichen die Verwendung eines herkömmlichen Kontaktsteckers 8a mit der erfindungsgemäßen Steckerbuchse 9. Ein herkömmlicher Kontaktstecker 8a wird vorzugsweise dann eingesetzt, wenn die Funktion der Defibrillation und/oder Kardioversion des Herzschrittmachers 1 nicht verwendet werden soll oder nicht benötigt wird. Derartige Kontaktstecker 8a werden üblicherweise mit Elektroden kombiniert, die nur für die Stimulation, also für eine reine Herzschrittmachertätigkeit verwendet werden.

Wie anhand der Figuren zu erkennen ist, ist der herkömmliche Kontaktstecker 8a zwar in die Steckerbuchse 9 einführbar, gelangt aber keinesfalls mit dem an seinem einzigen Steckerteil 20 ausgebildeten Kontaktbereich 12 lediglich in den Bereich des Gegenkontakts 13 und nicht in den Bereich des Gegenkontakts 14 der Steckerbuchse 9, der zur Übertragung von Defibrillations- und/oder Kardioversionsimpulsen auf eine dafür geeignete Elektrode entsprechend beschaltet ist.

Die Verwendung herkömmlicher Elektroden mit herkömmlichen Kontaktsteckern 8a mit dem Herzschrittmacher 1 ist also möglich, eine zu vermeidende Abgabe von Defibrillationsund/oder Kardioversionsimpulsen, die aufgrund der mangelnden Eignung der herkömmlichen, nur für die Stimulation vorgesehenen Elektrode zu Verletzungen am Herzen 5 führen könnte, jedoch ausgeschlossen.

Die gemäß Figur 1 an den externen Herzschrittmacher 1 angeschlossene Elektrode ist eine unipolare Elektrode 2a, die sich zur uni- und/oder monopolaren Stimulation und/oder Defibrillation und/oder Kardioversion eignet.

Für die Durchführung der Stimulation, der Defibrillation und der Kardioversion ist dabei eine zweite, anderweitig am oder im Körper, insbesondere am Rücken, eines Patienten anzubringende, Elektrode, die einen zweiten Pol oder Gegenpol bildet, zu verwenden.

So ist es möglich, den für die Stimulation und Defibrillation und für die Kardioversion notwendigen Strom von der Elektrode 2a auf das Herz 5 des Patienten und schließlich über die zweite, in den Figuren nicht dargestellte, außenseitig aufgebrachte Elektrode abzuleiten.

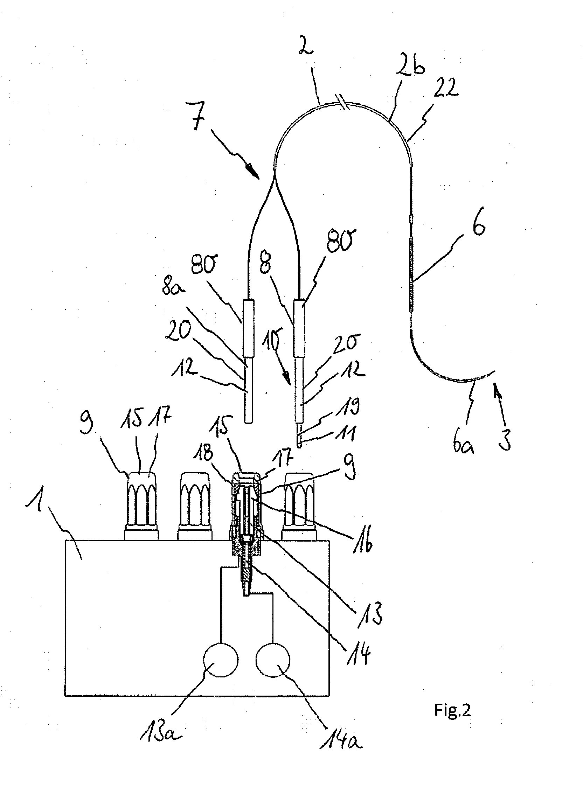

Figur 2 zeigt eine Elektrode 2b, die dadurch zur bipolaren Stimulation und/oder Defibrillation und/oder zur Kardioversion ausgebildet ist, dass sie wenigstens zwei Pole aufweist und dass die Elektrode 2b an ihrem proximalen Ende 7 für jeden Pol je einen Kontaktstecker 8 oder 8a und der Herzschrittmacher 1 für jeden der beiden Kontaktstecker 8 und 8a zumindest eine Steckerbuchse 9 aufweist.

Die beiden Pole dieser Elektrode 2b sind dabei an einem einzigen, an dem das Gewebe 4 des Herzens 5 kontaktierenden Wirkbereich am distalen Ende 3 der Elektrode 2b ausgebildet und in den Figuren nicht gesondert gekennzeichnet.

Der in den Figuren dargestellte Herzschrittmacher 1 weist zumindest vier einzelne Steckerbuchsen 9 auf, kann aber auch mit weiteren Steckerbuchsen 9 ausgestattet sein.

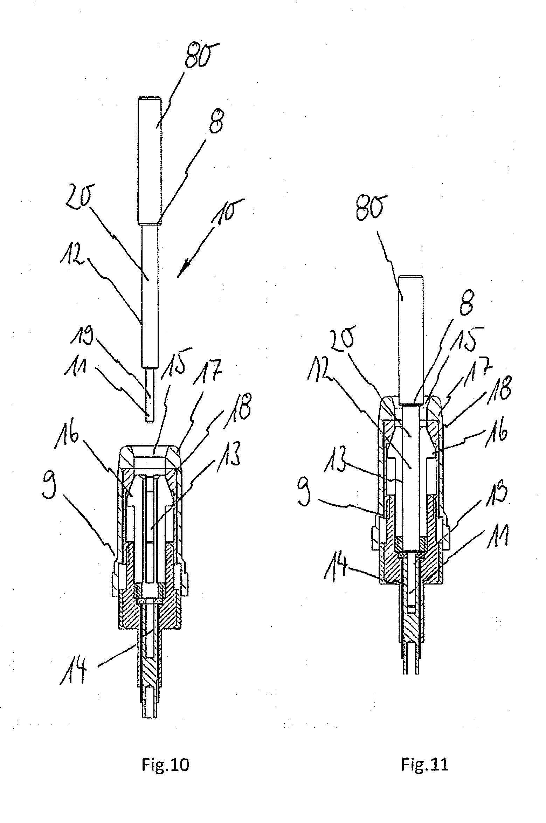

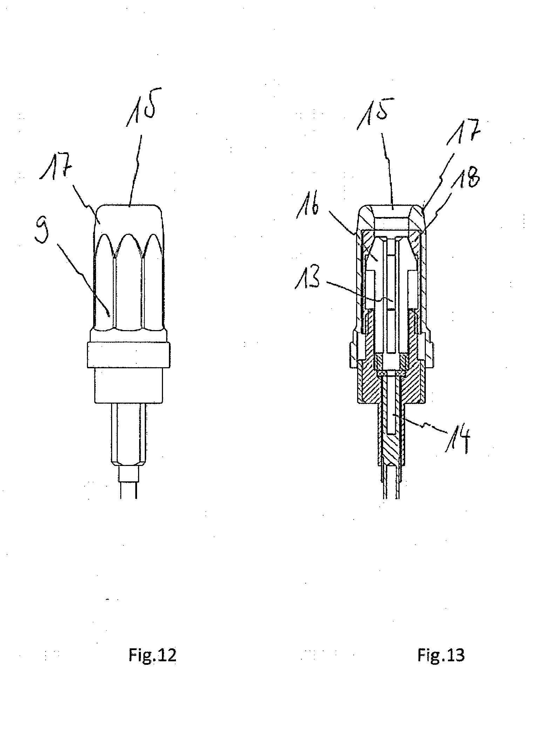

Sämtliche an dem Herzschrittmacher 1 ausgebildeten Steckerbuchsen 9 haben in ihrem Inneren die beiden unterschiedlich beschaltbaren und unterschiedlich geformten Gegenkontakte 13 und 14. Der Aufbau der Steckerbuchsen 9 ist besonders gut anhand der beiden Figuren 11 und 12 zu erkennen.

Gemäß Figur 2 hat die Elektrode 2b an ihrem proximalen Ende 7 zwei Kontaktstecker 8 und 8a. Von diesen beiden weist der Kontaktstecker 8 als Kontaktbereiche 11 und 12 die zwei zu den beiden Gegenkontakten 13 und 14 der Steckerbuchse 9 passenden Steckerteile 19 und 20 auf. Der Kontaktstecker 8 ist somit zweistufig ausgebildet und aufgrund seiner beiden unterschiedlich gestalteten Steckerteile 19 und 20 auch zur Übertragung eines Defibrillations- und/oder Kardioversionsimpulses auf die Elektrode 2b geeignet.

Bei dem zweiten Kontaktstecker 8a handelt es sich um einen herkömmlichen Kontaktstecker, der einstufig ausgebildet ist und dessen Steckerteil 20 in Gebrauchsstellung lediglich den Gegenkontakt 13 der Steckerbuchse 9 berührt und der als Gegenpol für die Defibrillation und/oder Kardioversion, also der Ableitung des Defibrillations- und/oder Kardioversionsimpulses dient, aber auch für eine Übertragung und/oder Ableitung von Stimulationsimpulsen genutzt werden kann.

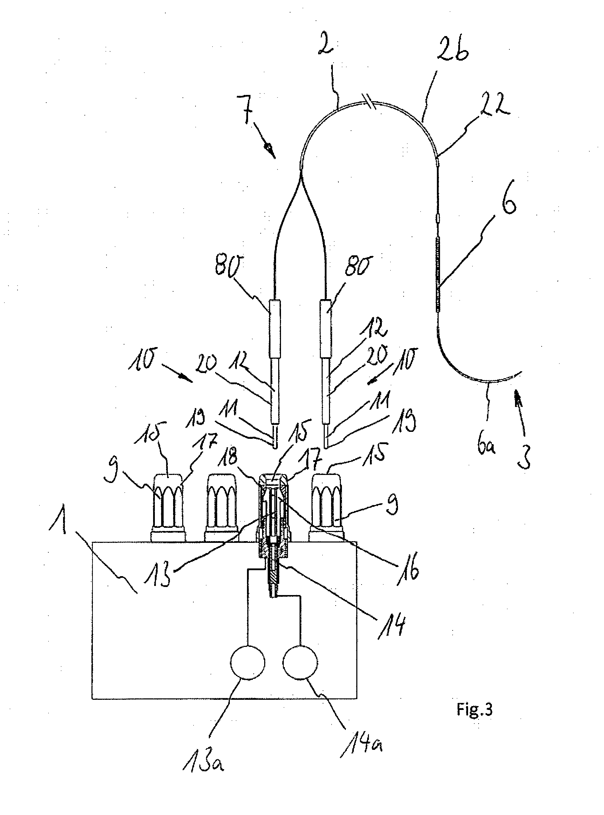

Figur 3 zeigt eine im Vergleich zu Figur 2 leicht abgewandelte Ausführungsform der bipolaren Elektrode 2b. Hier hat die Elektrode 2b an ihrem proximalen Ende 7 für jeden Pol je einen Kontaktstecker 8.

Beide Kontaktstecker 8 sind zweistufig ausgebildet und weisen jeweils zwei unterschiedlich gestaltete Steckerteile 19 und 20 auf, die die Kontaktbereiche 11 und 12 für die beiden Gegenkontakte 13 und 14 der Steckerbuchse 9 bilden.

Bei einer derartigen Elektrode 2b kann der Defibrillationsund/oder Kardioversionsimpuls sowohl über den einen als auch über den anderen der beiden Kontaktstecker 8 auf die Elektrode 2 übertragen werden.

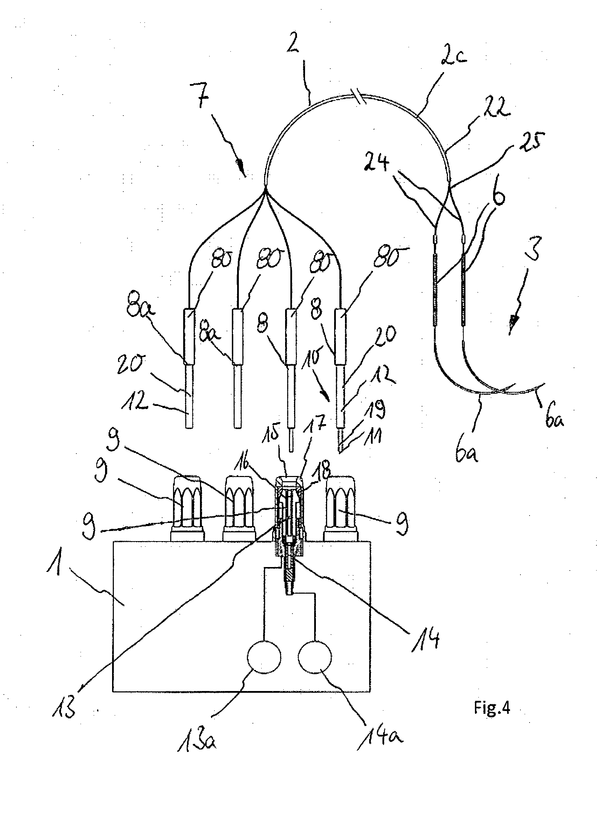

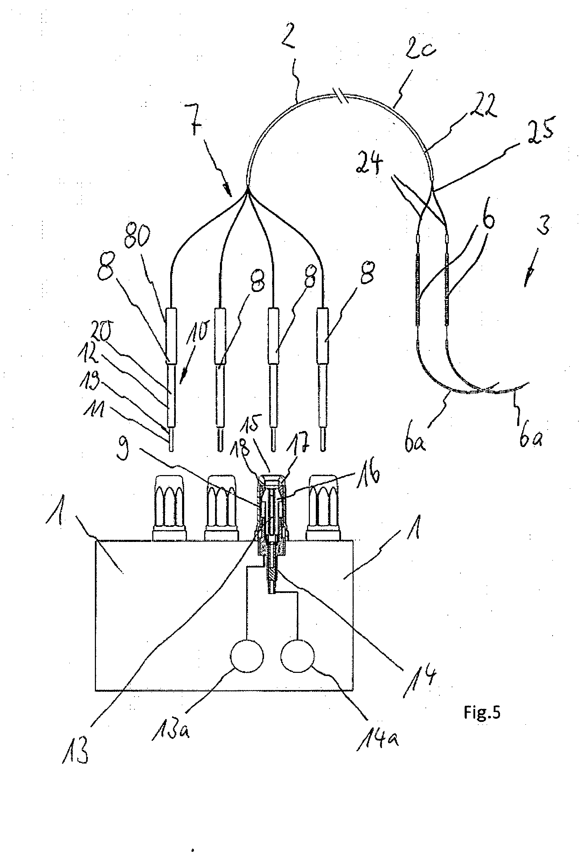

Die Figuren 4 und 5 zeigen eine weitere Ausführungsform einer erfindungsgemäßen Elektrode. Die gezeigte Elektrode 2c ist als quattripolare Elektrode ausgebildet und hat an ihrem proximalen Ende 7 für jeden Pol einen, also insgesamt vier Kontaktstecker 8 und 8a. An ihrem distalen Ende 3 weist diese Elektrode 2c zwei Herznadeln 6a auf, mit deren Hilfe die im Gewebe des Herzens 5 implantierbaren Bereiche 6 im Gewebe positioniert werden. Die beiden Bereiche 6 sind benachbart zu einer Verzweigung 25 der Elektrode 2c an jeweils einem separaten Elektrodenast 24 ausgebildet und weisen je zwei der insgesamt vier Pole auf.

Gemäß Figur 4 weisen die zwei Kontaktstecker 8 der vier Kontaktstecker jeweils die zweistufige Gestaltung mit zwei unterschiedlichen Steckerteilen 19 und 20 auf, während die beiden übrigen Kontaktstecker 8a nur über jeweils einen Steckerteil 20 verfügen, der zur Übertragung des Stimulationsimpulses auf die Elektrode 2 von dem ersten Gegenkontakt 13 der jeweiligen Steckerbuchse 9 eingerichtet ist.

Bei der in Figur 5 dargestellten Elektrode 2c weisen alle vier Kontaktstecker 8 je zwei unterschiedlich gestaltete Steckerteile 19 und 20 auf. Damit können alle Kontaktstecker 8 nicht nur für die Übertragung eines Stimulations-, sondern auch für eine Übertragung eines Defibrillations- und/oder Kardioversionsimpulses auf die Elektrode 2c benutzt werden.

Bei allen Ausführungsformen sei darauf hingewiesen, dass zur Übertragung eines Defibrillations- und/oder Kardioversionsimpulses auf die Elektrode 2, 2a, 2b, 2c, 2d zumindest ein Pol über einen zweistufig ausgebildeten Kontaktstecker 8 mit zwei unterschiedlich gestalteten Steckerteilen 19 und 20 verbunden sein muss, um den Defibrillations- und/oder Kardioversionsimpuls aus der Steckerbuchse 9 auf die Elektrode 2, 2a, 2b, 2c oder 2d zu übertragen.

Sämtliche in den Figuren gezeigten Kontaktstecker 8 sind entweder im Auslieferzustand mit der Elektrode 2, 2a, 2b, 2c oder 2d verbunden oder aber nachträglich mit der Elektrode 2, 2, 2a, 2b, 2c oder 2d verbindbar bzw. an diese anschließbar.

Bei den in den Figuren 1 bis 5 gezeigten Ausführungsbeispielen ist die Elektrode 2, 2a, 2b und 2c als Herzdraht 22 ausgebildet, der vorwiegend nach kardiochirurgischen Operationen direkt auf das Herz 5 genäht und durch eine unterhalb des Brustbeins eingebrachte Öffnung durch die Haut nach außen geführt wird, um an den externen Herzschrittmacher 1 angeschlossen zu werden.

Bei der in Figur 14 gezeigten Elektrode 2d handelt es sich um eine bipolare Elektrode, die an ihrem proximalen Ende 7 zwei Kontaktstecker 8 aufweist, von denen beide zweistufig ausgebildet sind und über jeweils zwei Steckerteile 19 und 20 verfügen. Diese Elektrode 2d ist dabei als ein intrakardial zuführbarer Katheter 23 ausgebildet, der über ein Blutgefäß von außen in das Innere des Herzens 5 vorgeschoben wird, um dort Stimulations- und/oder Kardioversions- und/oder Defibrillationsimpulse auf das Herz 5 des Patienten abzugeben.

Dabei muss für die Abgabe von Stimulations- und/oder Kardioversions- und/oder Defibrillationsimpulsen über den Wirkbereich 6 auf das Gewebe 4 des Herzens 5 kein unmittelbarer Berührkontakt zwischen Wirkbereich 6 und dem Gewebe 4 bestehen. Aufgrund der elektrischen Leitfähigkeit des Blutes und anderer Körperflüssigkeiten, kann die Übertragung der Impulse bereits dann erfolgen, wenn der Wirkbereich 6 nur an das Gewebe 4 angenähert ist, dieses aber noch nicht unmittelbar berührt. Der externe Herzschrittmacher 1 weist die wenigstens eine temporär mit dem Herzen 5 verbindbare Elektrode 2, 2a, 2b, 2c, 2d auf, an deren distalen Ende 3 der das Gewebe 4 des Herzens 5 kontaktierende Wirkbereich 6 und an deren proximalen Ende 7 wenigstens ein Kontaktstecker 8, 8a angeordnet sind. Die Elektrode 2, 2a, 2b, 2c, 2d ist für die Übertragung von Stimulations- und/oder Defibrillations- und/oder Kardioversionsimpulsen geeignet und zu diesem Zweck in die Steckerbuchse 9 am Herzschrittmacher 1 einsteckbar. Dabei weist der Kontaktstecker 8 den einen Kontaktbereich 11 an seinem Steckerteil 19 und den Kontaktbereich 12 an seinem Steckerteil 20 auf. Die Steckerbuchse 9 ist zu dem Kontaktstecker 8 passend ausgebildet und weist die beiden Gegenkontakte 13 und 14 auf, von denen der eine Gegenkontakt 13 zur Übertragung von Stimulationsimpulsen und der zweite Gegenkontakt 14 zur Übertragung von Defibrillations- und/oder Kardioversionsimpulsen auf den Kontaktstecker 8 eingerichtet ist. In eingesteckter Gebrauchsstellung gelangt nur der Kontaktbereich 11 an dem Steckerteil 19 mit dem für die Übertragung der Defibrillations- und/oder Kardioversionsimpulse vorgesehenen Gegenkontakt 14 in Berührung, sodass kein Defibrillationsund/oder Kardioversionsimpuls auf Elektroden, die für eine Defibrillation und/oder Kardioversion ungeeignet sind und lediglich einen einstufigen Kontaktstecker 8a aufweisen, übertragen werden kann.

## Patentansprüche

1. Externer Herzschrittmacher (1) mit wenigstens einer temporär mit einem Herzen verbindbaren Elektrode (2, 2a, 2b, 2c, 2d), an deren distalen Ende (3) zumindest ein ein Gewebe (4) des Herzens (5) eines Patienten in Gebrauchsstellung kontaktierender Wirkbereich (6) angeordnet ist, wobei die Elektrode (2, 2a, 2b, 2c, 2d) in Gebrauchsstellung mit ihrem proximalen Ende (7) über wenigstens einen Steckverbinder, bestehend aus zwei Teilen, nämlich aus einem Kontaktstecker (8, 8a) und aus einer Steckerbuchse (9), zur Übertragung von Stimulationsimpulsen von dem externen Herzschrittmacher (1) auf das Herz (5) des Patienten an den externen Herzschrittmacher (1) anschließbar oder angeschlossen ist, und wobei die Elektrode (2, 2a, 2b, 2c, 2d) zumindest an ihrem das Gewebe des Herzens (5) kontaktierenden Wirkbereich (6) eine für die Übertragung oder Abgabe eines Defibrillations- und/oder Kardioversionsimpulses ausreichend große Oberfläche hat, **dadurch gekennzeichnet, dass** der Kontaktstecker (8) des Steckverbinders einen ersten und einen zweiten elektrischen Kontaktbereich (11, 12) und die Steckerbuchse (9) des Steckverbinders, dazu passend, einen ersten und einen zweiten elektrischen Gegenkontakt (13, 14) aufweisen, dass die beiden Kontaktbereiche (11, 12) oder die beiden Gegenkontakte (13, 14) des an dem externen Herzschrittmacher (1) angeordneten Teils des Steckverbinders unterschiedlich beschaltbar oder beschaltet sind, sodass der eine der beiden Kontaktbereiche (11, 12) oder Gegenkontakte (13, 14) zur Übertragung von Stimulationsimpulsen und der andere der beiden Kontaktbereiche (11, 12) oder Gegenkontakte (13, 14) zur Übertragung von Defibrillations- und/oder Kardioversionsimpulsen auf die Elektrode (2, 2a, 2b, 2c, 2d) ausgebildet sind, und dass der externe Herzschrittmacher (1) mit einem Defibrillator und/oder Kardioverter verbunden ist und/oder einen Defibrillator und/oder einen Kardioverter enthält und/oder zur Abgabe von Defibrillationsund/oder Kardioversionsimpulsen eingerichtet ist.

2. Herzschrittmacher nach Anspruch 1, **dadurch gekennzeichnet, dass** der Kontaktstecker (8) des Steckverbinders an dem proximalen Ende (7) der Elektrode (2, 2a, 2b, 2c, 2d) und die Steckerbuchse (9) des Steckverbinders an dem externen Herzschrittmacher (1) angeordnet sind.

3. Herzschrittmacher nach Anspruch 1 oder 2, **dadurch gekennzeichnet, dass** die beiden unterschiedlich beschaltbaren oder beschalteten Gegenkontakte (13, 14) der Steckerbuchse (9) unterschiedlich und die beiden Kontaktbereiche (11, 12) des Kontaktsteckers (8) passend zu den Gegenkontakten (13, 14) geformt sind und/oder dass die beiden Kontaktbereiche (11, 12) des Kontaktsteckers (8) als zwei zu den beiden Gegenkontakten (13, 14) der Steckerbuchse (9) passende Steckerteile (19, 20) ausgebildet sind.

4. Herzschrittmacher nach einem der Ansprüche 1 bis 3, **dadurch gekennzeichnet, dass** der erste und der zweite Gegenkontakt (13, 14) der Steckerbuchse (9) in Einsteckrichtung des Kontaktsteckers (8,8a) hintereinander angeordnet sind und zwei unterschiedlich große Innenquerschnitte aufweisen.

5. Herzschrittmacher nach einem der Ansprüche 1 bis 4, **dadurch gekennzeichnet, dass** der erste Gegenkontakt (13) der Steckerbuchse (9) einen größeren Innenquerschnitt als der zweite Gegenkontakt (14) hat und als Kontaktfläche zur Übertragung von Stimulationsimpulsen auf die Elektrode (2, 2a, 2b, 2c, 2d) ausgebildet ist und dass der zweite, in Einsteckrichtung des Kontaktsteckers (8, 8a) benachbarte Gegenkontakt (14) der Steckerbuchse (9) einen kleineren Innenquerschnitt als der erste Gegenkontakt (13) hat und als Kontaktfläche zur Übertragung von Defibrillations- und/oder Kardioversionsimpulsen auf die Elektrode (2, 2a, 2b, 2c, 2d) ausgebildet sind.

6. Herzschrittmacher (1) nach einem der Ansprüche 1 bis 5, **dadurch gekennzeichnet, dass** der erste Gegenkontakt (13) mit dem größeren Innenquerschnitt in Gebrauchsstellung an eine Eintrittsöffnung (15) der Steckerbuchse (9) für den Kontaktstecker (8, 8a) angrenzend angeordnet ist und der zweite Gegenkontakt (14) mit dem kleineren Innenquerschnitt in Einsteckrichtung des Kontaktsteckers (8, 8a) dahinter folgt.

7. Herzschrittmacher (1) nach einem der Ansprüche 1 bis 6, **dadurch gekennzeichnet, dass** die Steckerbuchse (9) im ersten Gegenkontakt (13) eine Spannzangenform und/oder einen geschlitzten Bereich mit einzelnen Fingern aufweist, die durch eine Überwurfmutter (17) und einen konischen Bereich (18) zusammendrückbar sind und/oder im zweiten Gegenkontakt (14) Kontaktfedern aufweist und/oder dass ein in den zweiten Gegenkontakt (14) der Steckerbuchse (9) passender, im Querschnitt dünnerer erster Teil des Kontaktsteckers (8) geschlitzt und/oder elastisch verformbar ist, und dass der Kontaktstecker (8) in Gebrauchsstellung in der Steckerbuchse (9) mit wenigstens einem seiner Kontaktbereiche (11, 12) kraftschlüssig gehalten und/oder festgeklemmt ist.

8. Herzschrittmacher nach einem der Ansprüche 1 bis 7, **dadurch gekennzeichnet, dass** die beiden die Kontaktbereiche (11, 12) des Kontaktsteckers (8) bildenden und zu den beiden Gegenkontakten (13, 14) der Steckerbuchse (9) passenden Steckerteile (19, 20) in Einsteckrichtung des Kontaktsteckers (8,8a) hintereinander angeordnet und mit derart unterschiedlichen Querschnitten ausgebildet sind, dass der zweite Steckerteil (20) des Kontaktsteckers (8), der in eingesteckter Gebrauchsstellung den ersten Gegenkontakt (13) der Steckerbuchse (9) kontaktiert, den in axialer Richtung dahinter angeordneten, in eingesteckter Gebrauchsstellung den zweiten Gegenkontakt (14) der Steckerbuchse (9) kontaktierenden ersten Steckerteil (19) des Kontaktsteckers (8) zumindest bereichsweise radial überragt.

9. Herzschrittmacher nach einem der Ansprüche 1 bis 8, **dadurch gekennzeichnet, dass** der erste und der zweite Steckerteil (19, 20) des Kontaktsteckers (8) jeweils einen geschlossenen und/oder kreisrunden Querschnitt haben oder dass wenigstens einer der beiden Steckerteile (19, 20) des Kontaktsteckers (8) einen von einer Kreisform abweichenden Querschnitt hat, der insbesondere oval oder dreieckig oder viereckig oder mehreckig oder kreisförmig mit wenigstens einer Abflachung ist.

10. Herzschrittmacher nach einem der Ansprüche 1 bis 9, **dadurch gekennzeichnet, dass** einer der beiden Steckerteile (19, 20) des Kontaktsteckers (8) einen mehreckigen, insbesondere dreieckigen Querschnitt hat, also die Form eines Prismas aufweist, und der andere Steckerteil (19, 20) des Kontaktsteckers (8) einen runden Querschnitt hat, also zylindrisch ist.

11. Herzschrittmacher nach einem der Ansprüche 1 bis 10, **dadurch gekennzeichnet, dass** der zweite Steckerteil (20) des Kontaktsteckers (8) mit dem größeren Querschnitt einen kreisrunden Querschnitt aufweist, während der erste Steckerteil (19) des Kontaktsteckers (8) mit dem kleineren Querschnitt eine beliebige Querschnittsform aufweist, wobei die größte Abmessung des ersten, kleineren Steckerteils (19) von der Projektion des kreisrunden Querschnitts des zweiten, größeren Steckerteils (20) des Kontaktsteckers (8) umschlossen ist.

12. Herzschrittmacher nach einem der Ansprüche 1 bis 11, **dadurch gekennzeichnet, dass** die Längsmittelachsen der beiden Steckerteile (19, 20) des Kontaktsteckers (8) koaxial zueinander und/oder miteinander fluchtend und/oder parallel, insbesondere versetzt zueinander, verlaufend angeordnet sind.

13. Herzschrittmacher nach einem der Ansprüche 1 bis 12, **dadurch gekennzeichnet, dass** die Elektrode (2, 2a, 2b, 2c, 2d) dadurch zur bipolaren und/oder quattripolaren und/oder multipolaren Stimulation und/oder Defibrillation und/oder zur Kardioversion ausgebildet ist, dass sie wenigstens zwei und/oder vier und/oder mehr Pole aufweist und dass für jeden Pol der Elektrode (2, 2a, 2b, 2c, 2d) je ein Steckverbinder, bestehend aus einem Kontaktstecker (8,8a) und einer Steckerbuchse (9), vorgesehen ist.

14. Herzschrittmacher nach einem der Ansprüche 1 bis 13, **dadurch gekennzeichnet, dass** die Elektrode (2, 2a, 2b, 2c, 2d) an ihrem distalen Ende (3) wenigstens einen zweiten das Gewebe (4) des Herzens (5) eines Patienten kontaktierenden Wirkbereich (6) aufweist und/oder dass an jedem Wirkbereich (6) wenigstens ein Pol, insbesondere zwei Pole angeordnet sind.

15. Herzschrittmacher nach einem der Ansprüche 1 bis 14, **dadurch gekennzeichnet, dass** der Kontaktstecker (8,8a) mit der Elektrode (2, 2a, 2b, 2c, 2d) verbunden und/oder nachträglich mit der Elektrode (2, 2a, 2b, 2c, 2d) verbindbar und/oder an diese anschließbar ist.

16. Herzschrittmacher nach einem der Ansprüche 1 bis 15, **dadurch gekennzeichnet, dass** die Elektrode (2) als Herzdraht (22) oder als ein intrakardial zuführbarer Katheter (23) ausgebildet ist.
